# EUROPEAN PATENT SPECIFICATION

(11) **EP 3 881 871 B1**
(45) Date of publication and mention of the grant of the patent: **23.04.2025**
(21) Application number: 21167992.3
(22) Date of filing: 13.09.2016
(51) Int. Cl.: A61L 27/04, A61L 27/06, A61L 27/30, A61L 27/32, A61L 27/54, C25D 11/00

(54) **METHOD FOR TREATING A METAL SURFACE AND BODY, COMPRISING A TREATED METAL SURFACE**
VERFAHREN ZUR BEHANDLUNG EINER METALLOBERFLÄCHE UND KÖRPER MIT EINER BEHANDELTEN METALLOBERFLÄCHE
PROCÉDÉ DE TRAITEMENT D'UNE SURFACE MÉTALLIQUE ET DE CORPS, COMPRENANT UNE SURFACE MÉTALLIQUE TRAITÉE

(30) Priority: 21.09.2015 DE 102015115878
(43) Date of publication of application: 22.09.2021
(62) Divisional of application: 16770227.3
(73) Proprietor: AAP Implantate AG, 12099 Berlin (DE)
(72) Inventor: ELIEZER, Amir, 84965 Omer (IL); GASQUÈRES, Cyrille, 60323 Frankfurt (DE)
(74) Representative: Friderichs, Gunther

(56) References cited:
- WO-A1-2012/007181
- WO-A2-2010/139451
- US-A1- 2014 318 974

## Description

### Field of the Invention

The invention relates to a method for treating a metal surface and to a body having a metal surface. In particular, the invention relates to medical devices comprising a surface with antibacterial properties.

### Background of the Invention

There are various ways to apply functional layers onto metal surfaces. In particular, it is known to coat a metal surface with a silver comprising layer which has antibacterial properties.

However, it has been discovered that a silver coating releases silver ions, resulting in antibacterial properties, only for a short period of time.

Document WO 2010/139451 A2 shows a method of functionalizing a metal surface with nano silver particles by using a plasma electrolytic oxidation process.

This method results in a better release of silver ions. However, it has been discovered that implants comprising such a coating release silver ions in vivo only for a limited period of time which may not sufficient for all applications.

### Object of the Invention

Given this background, it is an object of the invention to provide a method for treating a metal surface and to provide a body having a treated metal surface which enables a controlled release of silver ions or another material.

### Summary of the Invention

The object of the invention is solved with a method for treating a metal surface and with a body having a treated surface according to one of the independent claims.

Preferred embodiments and modifications of the invention are set forth in the respective dependent claims.

The invention relates to a method for treating a metal surface.

In particular, the invention relates to the treatment of the metal surface of a medical device, for example an implant. However, the method can also be used to apply coatings, in particular antibacterial layers, onto handles, switches, control elements, etc., having a metal surface.

The body which has to be treated has a metal surface. Typically, a body made of a metal or a metal alloy is treated. However, it is also possible to use the invention for a dielectric body which is coated with a metal surface.

According to the invention, a colloid dispersed system is provided which comprises first particles. In particular, the colloid dispersed system comprises silver particles.

The particles can have a size between 5 nm and 1 µm. Preferably, particles with an average particle size below 200 nm are used. The particle size can be determined by electron microscopy. With respect to particles which are not spherically shaped, the average particle size, according to the invention, is defined as the mean value of length, width and height.

The particles are, for example, available as aqueous dispersion comprising the particles and at least one electrolyte. In particular, when using metal particles, the particles can be provided with a stabilizing agent, in particular with a polymer-based stabilizing agent, in order to prevent the particles from sedimentation.

According to the invention, a first layer is applied by immersing the metal surface into the electrolyte and treating the metal surface with a plasma electrolytic oxidation process wherein the first particles are deposited on the surface.

The plasma electrolytic oxidation process can be achieved by applying a voltage above a specific value which results in generating plasma in the electrolytic bath on the metal surface. The metal surface is oxidized and, simultaneously, the particles are embedded in and on the surface.

Preferably, an AC-voltage is used, in particular an asymmetric AC-voltage. This results in a more homogeneous layer.

The layer which is applied by using the plasma electrolytic oxidation process typically has a thickness of 3 µm to 50 µm.

The electrolytic bath can comprise further materials, in particular also larger particles without colloidal properties.

According to the invention, a second layer is applied. The second layer is be applied upon the first layer.

The second layer can influence the properties of the first layer, in particular with respect to ion release. In particular, the second layer can be used as a coating upon the first layer in order to decrease the initial release of ions. For example, a biodegradable layer can be used.

A layer which is applied below the first layer, for example, a layer with a material which has a higher standard electrode potential (a more noble metal) can increase the release of ions.

A second layer can be applied also adjacent to the first layer, thereby providing a body comprising areas with different coatings.

Also, the second layer is be applied by a plasma electrolytic oxidation process, in particular by performing the plasma electrolytic oxidation process in an electrolytic bath comprising colloid dispersed particles.

One embodiment of the invention relates to the application of a multilayer system which is applied by plasma electrolytic oxidation process. It has been discovered that it is possible to apply two different layers by using the plasma electrolytic oxidation process on each layer.

Such layers can be applied, for example, by using different electrolytical baths, for example, using baths having different particles and/or a different particle concentration.

According to one embodiment of the invention, the metal surface is part of a body which comprises at least two different metals or metal alloys.

According to one embodiment of the application, hollow spheres are provided as first or second particles.

Hollow spheres, which are commercially available, could also be used in a plasma electrolytic oxidation process and can be deposited onto the surface of the body to be treated.

The hollow spheres can serve as a reservoir for pharmaceutical substances. The substrate, which has a surface comprising hollow spheres, can be, for example, dipped into a bath comprising said pharmaceutical substances.

The body can comprise, in particular, titanium, stainless steel, aluminum, copper, zinc and/or magnesium.

In particular, the body can comprise areas which are biodegradable.

The invention relates to a body, in particular to a medical device, for example an implant, which comprises a first layer with silver particles and a second layer which comprises a calcium phosphate, in particular, a hydroxyapatite.

The second layer can comprise also magnesium as biodegradable material.

In further, the body can comprise a layer comprising a more noble material which is applied below the first layer which comprises silver particles in order to increase the release of silver ions.

According to one embodiment of the invention, the body comprises two layers with silver particles wherein a further layer is placed between the layers with silver particles.

For example, the uppermost layer can comprise silver particles and can be embodied as a biodegradable layer by also comprising magnesium, for example.

This layer degrades and releases silver ions.

Then, a layer follows which comprises calcium phosphate, in particular hydroxyapatite, and magnesium which can be applied also by the plasma electrolytic oxidation process, for example, and which degrades slowly, thereby protecting the further layer comprising silver particles which is placed below the layer comprising calcium phosphate.

The invention also relates to a method for treating a metal surface, in particular as described before.

According to this embodiment of the invention, a colloid dispersed system is provided which comprises first particles and a first layer is applied by treating the metal surface with a plasma electrolytic oxidation process and thereby depositing the particles on the surface. Then, a second layer is applied by treating the metal surface with a plasma electrolytic oxidation process and thereby depositing second particles on the surface.

According to the invention, the particle concentration in said first layer is different as the particle concentration in said second layer.

For example, two different electrolytic bathes are used with a different concentration of particles (silver particles for example). An uppermost layer with a higher concentration of particles results in a high release of silver ions in an initial phase after insertion of an implant for example. Then, a second layer follows with a lower concentration of silver particles.

A further embodiment of the invention relates to a method for treating a metal surface, in particular to a method as described before.

This method also relates to a plasma electrolytic oxidation process wherein a colloid dispersed system is provided which comprises first particles and wherein the first particles are deposited on the surface.

According to the invention, silver particles are provided as said first particles and also a biodegradable material is applied onto the metal surface.

In particular, the biodegradable material is applied so that the layer which comprises the silver particles also comprises the biodegradable material.

Magnesium, for example, can be used as biodegradable material.

For example, a layer of elemental magnesium can be deposited, for instance, by sputtering or electron beam deposition.

Then, the body comprising the magnesium layer is treated with the plasma electrolytic oxidation process, thereby forming a layer which comprises magnesium oxide and silver particles.

In further, it is possible to deposit also calcium phosphate particles, for example, hydroxyapatite particles.

The layer can also comprise a further metal oxide, for example, an oxide which is generated by oxidizing the metal of the body which is treated, for example, titanium oxide.

In one embodiment, a layer is applied which comprises magnesium oxide, silver particles and a further metal oxide. By adapting the amount of said components, it is possible to achieve a controlled release of silver ions.

According to a further aspect of the invention, a metal surface is treated by using a colloid dispersed system comprising first particles of a first metal and second particles of a second metal, which is different from the first metal.

The surface is treated by using a plasma electrolytic oxidation, wherein first and second particles are deposited on the metal surface.

The first particles preferably comprise metal which shall be released, in particular silver.

By producing a surface which comprises second particles of a different metal, it is possible to achieve a controlled release of ions, in particular silver ions. As a second metal preferably a more noble metal, in particular gold, is used.

The ratio (in weight percentage) of the first metal to the second metal is preferably in the range between 1 to 10 and 10 to 1.

As metal particles preferably nanoparticles are used, in particular particles with an average particle size of less than 200 nm, preferably of less than 50 nm.

The metal particles for all embodiments of the invention can be added to the electrolytic bath as dispersion comprising the metal particles and a stabilizer.

Preferably an aqueous suspension comprising the metal particles is used.

As a stabilizer, a nonionic surfactant can be used, in particular in a nonionic surfactant selected from the group consisting of polyoxyethylene-mono-alkyl acid ester, polyoxypropylene-mono-alkyl acid ester, polyoxyethylene-di-alkyl-acid ester, polyoxypropylene-di-alkyl acid ester, polyoxyethylene-tri-alkyl acid ester, and polyoxypropylene-tri-alkyl acid ester. The stabilizer prevents the metal particles from sedimentation.

### Brief Description of the Drawings

The subject matter of the invention will now be described in more detail with reference to exemplary embodiments as illustrated in the drawings Fig. 1 to Fig. 8.
Fig. 1 to Fig. 8 show schematically various embodiments of the treated metal surface of a body according to the invention.
Fig. 9 schematically illustrates an embodiment of applying a multilayer system according to the invention onto the substrate.

### Detailed Description of the Drawings

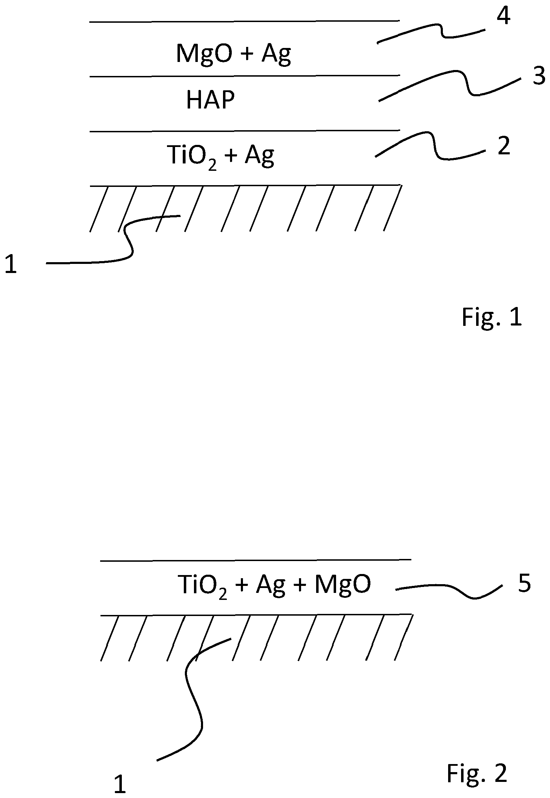

Fig. 1 shows a schematic sectional view of a substrate 1 comprising a metal surface.

The substrate is in particular embodied as a medical device, for example, as an implant which shall be inserted in the body of a human or an animal.

However, the invention can also be used for other metal surfaces which come into contact with humans or animals.

Onto the substrate 1 a first layer 2 is applied which comprises silver particles and titanium oxide.

The layer 2 is applied by a plasma electrolytic oxidation process, thereby converting the titanium surface of the substrate 1 into titanium oxide and simultaneously embedding silver particles.

Onto the first layer 2, a second layer 3 is applied which comprises hydroxyapatite. This layer can also be applied by using a plasma electrolytic oxidation process. For example, an electrolytic bath can be used which comprises colloid dispersed hydroxyapatite and also apatite powder. The apatite powder preferably consists of agglomerated nanoparticles.

Onto said hydroxyapatite layer a further layer is applied which comprises magnesium oxide and silver.

This layer can also be applied by plasma electrolytic oxidation. As a first step, a magnesium layer can be applied, for example, by using a sputtering process. Then, the magnesium surface is immersed by the plasma electrolytic oxidation process, wherein the bath, in which the oxidation is performed, comprises colloidal silver particles which are dispersed in the bath.

The magnesium comprising layer 4 degrades, for example, after the insertion of the implant.

The hydroxyapatite comprising layer 3 achieves a better ingrowth if the substrate is used as an implant.

The titanium oxide and silver comprising layer 2 results in a surface with biocidal properties for a long time period.

Fig. 2 shows an embodiment wherein the substrate 1 comprises a single layer only.

The single layer 5 comprises magnesium oxide, silver and a further metal oxide, in this case titanium oxide.

Such a layer can be produced by depositing a thin magnesium layer onto the substrate 1.

Then, a plasma electrolytic oxidation process is performed, thereby converting the magnesium layer into magnesium oxide. Also, the metal surface of the substrate 1 is at least partially converted into a metal oxide, in this case into titanium oxide.

The ratio of titanium oxide and magnesium oxide can be adjusted by varying the thickness of the deposited magnesium layer.

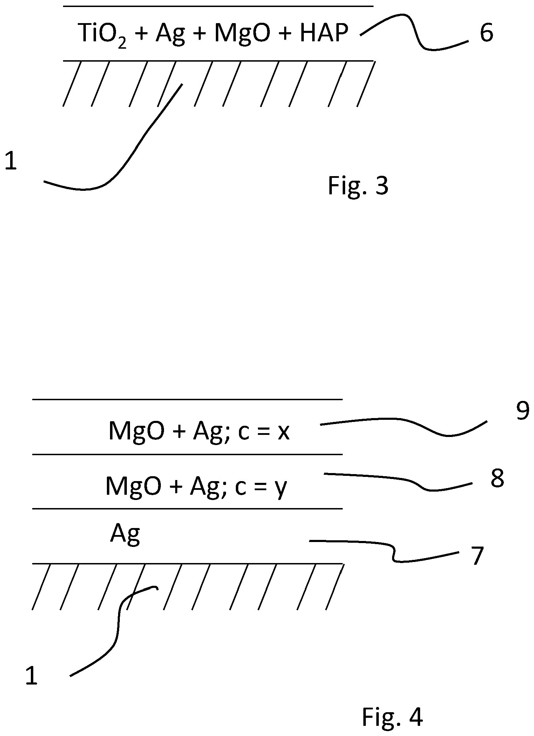

Fig. 3 shows a further embodiment wherein a layer 6 is applied onto the substrate.

This embodiment corresponds with Fig. 2, with the difference that the layer also comprises hydroxyapatite.

In this embodiment, the electrolytic bath for the plasma electrolytic oxidation process also comprises hydroxyapatite, preferably in the form of colloid dispersed particles, and additional hydroxyapatite powder with an average particle size between 10 µm and 100 µm.

Fig. 4 shows a further embodiment wherein onto the substrate a first layer 7 comprising silver particles is applied.

Then, the layers 8 and 9 which comprise magnesium oxide are applied. The layer 9 has a different concentration of silver particles than the layer 8. The layers 8 and 9 will be degraded. By varying the amount of magnesium oxide and silver particles, it is possible to have a controlled release of silver ions, also over a long period of more than one week in vivo.

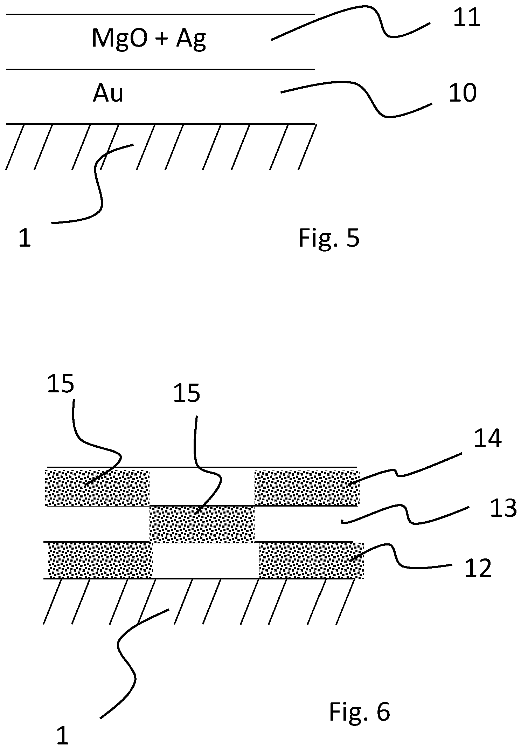

Fig. 5 shows a further embodiment of the invention.

Onto the substrate 1, as a first layer, a gold layer 10 is applied. A gold layer can, for example, be sputtered. The layer can be very thin.

Onto this layer a magnesium oxide and silver particles comprising layer is applied. Also this layer is achieved by depositing a magnesium layer first and by performing a plasma electrolytic oxidation process in a further step.

Due to the higher electrical potential of gold, the release of silver ions of the layer 11 is increased.

Fig. 6 shows a further embodiment of the invention wherein the substrate 1 comprises the layers 12, 13, and 14.

The layers 12, 13, and 14 comprise particles 15, for example silver particles, in different regions.

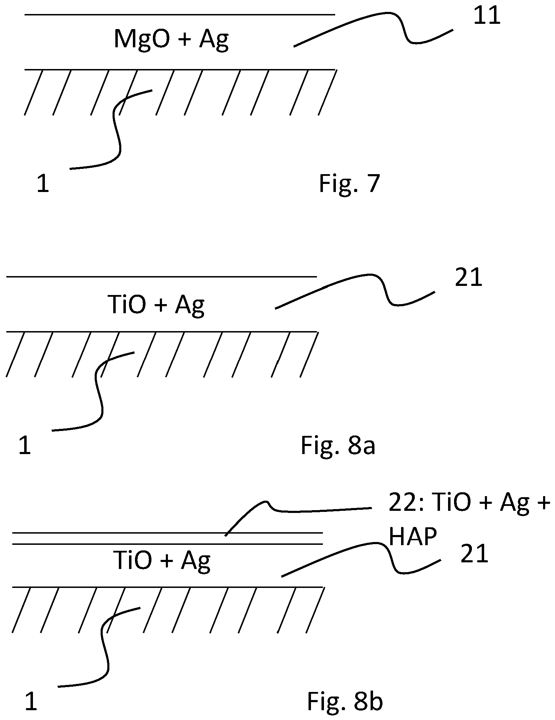

Fig. 7 shows a further embodiment wherein the substrate 1 comprises a layer 11, consisting of magnesium oxide and silver particles.

This layer degrades, thereby releasing silver ions for a long period.

Referring to Fig. 8 a and Fig. 8 b, a further embodiment of the invention shall be described.

As first step, shown in Fig. 8 a, a titanium implant is provided with a titanium oxide layer 21 which comprises silver particles. This layer has a thickness between 20 and 100 µm and is applied by using a PEO process.

Then, as next step shown in Fig. 8 b, the surface of the layer 21 is converted by a PEO process with an electrolytic bath comprising hydroxyapatite particles, thereby forming a thinner layer 22 comprising also hydroxyapatite.

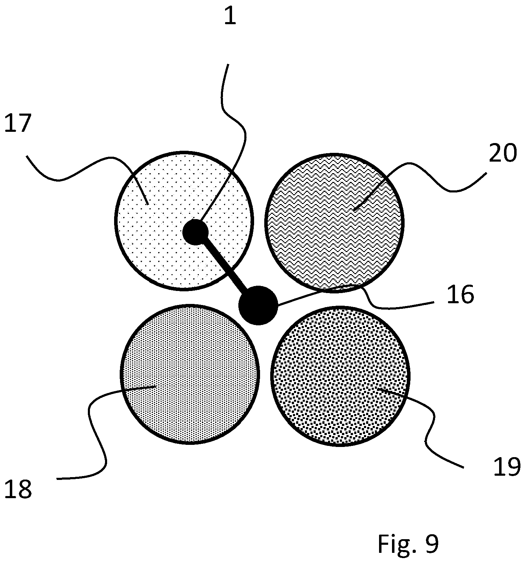

Fig. 9 shows schematically how a substrate 1 can be coated with a multilayer system. The substrate 1 can be dipped by a robot 16 into different electrolytic baths 17 to 20.

The first bath 17, for example, can comprise silver particles in a first concentration.

The second bath 18 can comprise silver particles in a different concentration, for example.

Then, the substrate 1 can be dipped into the third bath 19 and also a plasma electrolytic oxidation process is performed wherein hydroxyapatite particles are inserted into the bath. As a next coating it might be possible to dip the substrate 1 into a polymer bath 20.

By combining a plasma electrolytic oxidation process with applying a further layer, in particular by applying additionally a biodegradable material, it is possible to achieve specific functional properties of biocidal layers or layer systems, in particular to achieve a controlled release of silver ions.

## Claims

1. A method for treating a metal surface, in particular of a medical device, comprising the following steps:
- providing a colloid dispersed system comprising first particles;
- applying a first layer by a plasma electrolytic oxidation wherein the first particles are deposited on the surface;
- applying a second layer upon the first layer, wherein the second layer is applied by providing a colloid dispersed system comprising second particles and by converting the surface by a plasma electrolytic oxidation wherein the second particles are deposited.

2. The method for treating a metal surface according to claim 1, wherein a multilayer system is applied by plasma electrolytic oxidation.

3. The method for treating a metal surface according to one of the preceding claims, wherein a first layer comprising the first particles is applied onto a first area of the metal surface and a second layer comprising second particles is applied onto a second area of the metal surface.

4. The method for treating a metal surface according to one of the preceding claims, wherein silver particles are provided as said first or as second particles.

5. The method for treating a metal surface according to one of the preceding claims, wherein at least two layers with a different concentration of particles are applied.

6. The method for treating a metal surface according to one of the preceding claims, wherein at least one layer with a gradually increasing or decreasing concentration of particles is applied.

7. The method for treating a metal surface according to one of the preceding claims, wherein calcium phosphate particles, in particular hydroxyapatite particles, are provided as said first or said second particles.

8. The method for treating a metal surface according to one of the preceding claims, wherein said first or said second layer comprises a biodegradable material, in particular magnesium.

9. The method for treating a metal surface according to one of the preceding claims, wherein hollow spheres are provided as said first or said second particles.

10. The method for treating a metal surface according to one of the preceding claims, wherein said metal surface is part of a body, comprising at least two different metals or alloys, in particular titanium, stainless steel and/or magnesium.

11. Body, in particular implant, comprising a metal surface, treated with a method according to one of the preceding claims.

12. The body according to the preceding claim, comprising a first layer with silver particles and at least on second layer, in particular a calcium phosphate and/or magnesium comprising layer and/or a layer comprising a more noble material.

13. The body according to the preceding claims, comprising at least two layers with silver particles, wherein a further layer, in particular a layer comprising calcium phosphate particles is placed between the layers with silver particles.

## Patentansprüche

1. Verfahren zur Behandlung einer Metalloberfläche, insbesondere von einer medizinischen Vorrichtung, umfassend folgende Schritte:
- Bereitstellen eines kolloid dispersen Systems, welches erste Partikel umfasst;
- Aufbringen einer ersten Schicht mittels plasmaelektrolytischer Oxidation, wobei die ersten Partikel auf der Oberfläche abgeschieden werden;
- Aufbringen einer zweiten Schicht auf die erste Schicht, wobei die zweite Schicht aufgebracht wird durch Bereitstellen eines kolloid dispersen Systems, welches zweite Partikel umfasst und durch Umwandeln der Oberfläche durch eine plasmaelektrolytische Oxidation, wobei die zweiten Partikel abgeschieden werden.

2. Verfahren zur Behandlung einer Metalloberfläche, nach Anspruch 1, wobei ein Mehrschichtsystem durch plasmaelektrolytische Oxidation aufgebracht wird.

3. Verfahren zur Behandlung einer Metalloberfläche nach einem der vorstehenden Ansprüche, wobei eine erste Schicht, welche die ersten Partikel umfasst, auf einen ersten Bereich der Metalloberfläche aufgebracht wird und wobei eine zweite Schicht, welche zweite Partikel umfasst, auf einen zweiten Bereich der Metalloberfläche aufgebracht wird.

4. Verfahren zur Behandlung einer Metalloberfläche nach einem der vorstehenden Ansprüche, wobei Silberpartikel als die zweiten Partikel bereitgestellt werden.

5. Verfahren zur Behandlung einer Metalloberfläche nach einem der vorstehenden Ansprüche, wobei mindestens zwei Schichten mit einer unterschiedlichen Konzentration von Partikeln aufgebracht werden.

6. Verfahren zur Behandlung einer Metalloberfläche nach einem der vorstehenden Ansprüche, wobei zumindest eine Schicht mit einer graduell zunehmenden oder abnehmenden Konzentration von Partikeln aufgebracht wird.

7. Verfahren zur Behandlung einer Metalloberfläche nach einem der vorstehenden Ansprüche, wobei Calciumphosphatpartikel, insbesondere Hydroxylapatit-Partikel, als erste oder zweite Partikel bereitgestellt werden.

8. Verfahren zur Behandlung einer Metalloberfläche nach einem der vorstehenden Ansprüche, wobei die erste oder zweite Schicht ein biodegradierbares Material, insbesondere Magnesium, umfasst.

9. Verfahren zur Behandlung einer Metalloberfläche nach einem der vorstehenden Ansprüche, wobei Hohlkugeln als erste oder zweite Partikel bereitgestellt werden.

10. Verfahren zur Behandlung einer Metalloberfläche nach einem der vorstehenden Ansprüche, wobei die Metalloberfläche Teil eines Körpers ist, welcher zumindest zwei verschiedene Metalle oder Legierungen umfasst, insbesondere Titan, Edelstahl und/oder Magnesium.

11. Körper, insbesondere Implantat, umfassend eine Metalloberfläche, welche mit einem Verfahren nach einem der vorstehenden Ansprüche behandelt ist.

12. Körper nach dem vorstehenden Anspruch, wobei der Körper eine erste Schicht mit Silberpartikeln und zumindest eine zweite Schicht umfasst, insbesondere ein Calciumphosphat, eine Magnesium enthaltende Schicht und/oder eine Schicht, welche ein edleres Material umfasst.

13. Körper nach dem vorstehenden Anspruch umfassend zumindest zwei Schichten mit Silberpartikeln, wobei eine weitere Schicht, insbesondere eine Schicht, welche Calciumphosphatpartikel umfasst, zwischen den Schichten mit Silberpartikeln angeordnet ist.

## Revendications

1. Procédé de traitement d'une surface métallique, en particulier d'un dispositif médical, comprenant les étapes suivantes:
- fournir un système colloïdal dispersé comprenant des premières particules;
- appliquer une première couche par une oxydation électrolytique au plasma, dans laquelle les premières particules sont déposées sur la surface;
- appliquer une seconde couche sur la première couche, la seconde couche étant appliquée en fournissant un système colloïdal dispersé comprenant des secondes particules et en convertissant la surface par une oxydation électrolytique au plasma, dans laquelle les secondes particules sont déposées.

2. Procédé de traitement d'une surface métallique selon la revendication 1, dans lequel un système multicouche est appliqué par oxydation électrolytique au plasma.

3. Procédé de traitement d'une surface métallique selon l'une quelconque des revendications précédentes, dans lequel une première couche comprenant les premières particules est appliquée sur une première zone de la surface métallique et une seconde couche comprenant des secondes particules est appliquée sur une seconde zone de la surface métallique.

4. Procédé de traitement d'une surface métallique selon l'une quelconque des revendications précédentes, dans lequel des particules d'argent sont fournies en tant que lesdites premières ou en tant que des secondes particules.

5. Procédé de traitement d'une surface métallique selon l'une quelconque des revendications précédentes, dans lequel au moins deux couches ayant une concentration différente de particules sont appliquées.

6. Procédé de traitement d'une surface métallique selon l'une quelconque des revendications précédentes, dans lequel au moins une couche est appliquée, dans laquelle une concentration de particules augmente ou diminue progressivement.

7. Procédé de traitement d'une surface métallique selon l'une quelconque des revendications précédentes, dans lequel des particules de phosphate de calcium, en particulier des particules d'hydroxyapatite, sont fournies en tant que lesdites premières ou lesdites secondes particules.

8. Procédé de traitement d'une surface métallique selon l'une quelconque des revendications précédentes, dans lequel ladite première ou ladite deuxième couche comprend un matériau biodégradable, en particulier du magnésium.

9. Procédé de traitement d'une surface métallique selon l'une quelconque des revendications précédentes, dans lequel des sphères creuses sont fournies en tant que lesdites premières ou lesdites secondes particules.

10. Procédé de traitement d'une surface métallique selon l'une quelconque des revendications précédentes, dans lequel ladite surface métallique fait partie d'un corps comprenant au moins deux métaux ou alliages différents, en particulier du titane, de l'acier inoxydable et/ou du magnésium.

11. Corps, en particulier implant, comprenant une surface métallique traitée par un procédé selon l'une quelconque des revendications précédentes.

12. Corps selon la revendication précédente, comprenant une première couche avec des particules d'argent et au moins une seconde couche, en particulier une couche comprenant du phosphate de calcium et/ou du magnésium, et/ou une couche comprenant un matériau plus noble.

13. Corps selon les revendications précédentes, comprenant au moins deux couches avec des particules d'argent, dans lequel une couche supplémentaire, en particulier une couche comprenant des particules de phosphate de calcium, est disposée entre les couches avec des particules d'argent.
